# EUROPEAN PATENT APPLICATION

(11) **EP 4 813 332 A1**
(43) Date of publication of application: **30.09.2026**
(21) Application number: 25167085.7
(22) Date of filing: 28.03.2025
(51) Int. Cl.: A61B 34/10, A61B 34/00, A61B 18/00

(54) **OPTIMAL PATH DETERMINATION IN ABLATION NEEDLE GUIDANCE**

(71) Applicant: Siemens Healthineers AG, 91301 Forchheim (DE)
(72) Inventor: CHANDRA SEKHAR, N., 560100 Bangalore (IN)
(74) Representative: HKW Intellectual Property PartG mbB

(57) **Abstract**

A system (1) for determining a number N1 of optimal paths (2a - 2c) for a lesion ablation treatment of a lesion (3) of a patient, with N1 ≥ 1, the system (1) comprising: a medical imaging unit (4) for acquiring a medical image (5) of the patient; a computer-implemented path determination unit (6) for an automatic determination of the N1 optimal paths (2a - 2c) based on the acquired medical image (5); and a user interface (7) for outputting the determined N1 optimal paths (2a - 2c) to a user of the system (1).

## Description

The present invention relates to a system for determining a number N1 of optimal paths for a lesion ablation treatment of a lesion of a patient, a method for using the said system and a computer program product.

Percutaneous tumor ablation comprises a range of techniques in which the tumor tissue is destroyed using needles inserted through the skin. In the vast majority of techniques, the tumor is destroyed with various types of needles placed in or around the tumor. The most commonly used techniques use heat (for example radiofrequency ablation [RFA] or microwave ablation [MWA]) or use cold (cryoablation). There are also non-thermal techniques that use an electric current, for example. In irreversible electroporation [IRE], high-voltage electric shocks are used to penetrate the cell membranes and induce cell death.

The aim of percutaneous tumor ablation is to destroy the tumor without surgery. Percutaneous tumor ablation is suitable for the treatment of a variety of benign and malignant tumors, including liver, kidney, lung and bone tumors. In selected patients, it is also used to treat other organs such as the adrenal glands or pancreas.

The procedure may be performed in a Computed Tomography [CT] room under anesthesia. The patient may be placed on a CT table and the patient's skin will be disinfected and sterilely covered. The ablation needle is then inserted into the tumor under ultrasound or CT control. As soon as the physician is sure that the catheter is in the right place, the ablation is started. Once the ablation is complete, the needle is removed, and the complete destruction of the lesion is confirmed by further CT scans.

Still, it represents a challenge to determine the right placement of the needle inside the patients' body. In particular, it is still a challenge to determine a suitable path for the needle through the patient's body in order to reach the tumor or other lesion without damaging or harming other parts of the patient's body, for example, the skeleton, the aorta, the heart, or others. Nowadays, the determination of a suitable path is performed manually in a time-consuming and/or error-prone manner. Hence, there is still a need for a method for an improved determination of the needle path.

Therefore, it is one objective of the present invention to improve the determination of the needle path.

According to a first aspect, a system for determining a number N1 of optimal paths for a lesion ablation treatment of a lesion of a patient is suggested, with N1 ≥ 1. The system comprises a medical imaging unit for acquiring a medical image of the patient, a computer-implemented path determination unit for an automatic determination of the N1 optimal paths based on the acquired medical image, and a user interface for outputting the determined N1 optimal paths to a user of the system.

The lesion can be a tumor or another disease of the liver, lung, kidney, bone, or other part of the patient. The lesion may be concentrated in only one location inside the patient's body or be spread, thus being present at various locations inside the patient's body. In case multiple lesions exist, they can either be treated subsequently and/or simultaneously. In the following, the term "lesion" can represent a single lesion or a spread lesion with multiple locations.

In the following, the term "path" can be understood as a line through the patient's body along which a needle for tumor ablation treatment is moved through the patient's body in order to treat the lesion. Hereby, the path may represent a straight line, a bent or curved line, or other shapes suitable for tumor ablation treatment. The N1 optimal paths can be understood as the most beneficial paths for tumor ablation treatment with the patient in the light of a possible damage to the patient's body, in particular, the possible damage of organs and/or tissue of the body by the needle by moving the needle through the patient's body, the respective required treatment time, the length of the respective path, or other criteria. The N1 optimal paths can represent a single path or a number of paths of equal and/or comparable benefit regarding a placement of the needle inside the lesion.

By determining the N1 optimal paths, the user of the system can be provided with the information along which path the needle has to be moved through the patient's body in order to treat the lesion while requiring the least treatment time and/or damaging the patient's body the least.

The user of the system may be a physician, technician, or other medical expert. The user can interact with the system using the user interface which may be a computer, a laptop, a tablet, a mobile device and/or input and/or output units of the before-mentioned devices.

The user interface can comprise a graphical user interface, for example a monitor, a display, a mobile device, or other devices capable of visualizing graphical information or textual information.

The medical imaging unit can be a computer tomography [CT] unit, an X-ray unit, or other units capable of acquiring medical images. Hereby, the medical image may be a two-dimensional slice through the patient's body and/or a three-dimensional illustration or representation of a part and/or of the whole patient's body. The medical image can comprise an illustration of the skin of the patient, the organs, the skeleton and/or the lesion.

In the following, the term "computer-implemented" can be understood as being implemented as a computer program product which can be run on a computer, a server, or on other devices. Alternatively, the computer-implemented path determination unit can also be implemented using solely hardware components.

The term "automatic" can be understand in such a way that the system is capable of providing the user with the N1 optimal paths without any additional effort or intervention of the user. Hence, the N1 optimal paths can be derived directly based on the acquired medical image.

According to an embodiment, the user interface comprises an input unit which is configured to receive an ablation needle configuration from the user, wherein the path determination unit further comprises a generic shape generation unit for generating a generic three-dimensional shape based on the received ablation needle configuration. In particular, the generic three-dimensional shape represents an idealized impact region for the lesion ablation treatment, wherein the generic shape generation unit is configured to determine the size and/or the shape of the generic three-dimensional shape depending on the received ablation needle configuration.

In the following, the ablation needle configuration can be understood as the specific technique used for the lesion ablation treatment, for example radio frequency ablation, microwave ablation, or cryoablation, defining both the size and diameter of the ablation needle as well as the strength of the lesion ablation treatment chosen in order to treat the lesion. The generic three-dimensional shape can be a sphere, ellipsoid, or any other three-dimensional shape which can represent an impact zone of the lesion ablation treatment. The impact zone represents the part, region or section of the patient's body where tissue is affected or destroyed by the lesion ablation treatment. In other words, the generic three-dimensional shape models the part, region or section of the patient's body, which will be affected by the lesion ablation treatment. Hence, the generic three-dimensional shape shall be of a respective shape and/or size in order to include the lesion of the patient. Therefore, the user interface can be configured to provide the user with a visual and/or textual feedback whether the whole lesion is included within the generic three-dimensional shape. Otherwise, a respective change of the ablation needle configuration can be suggested to the user. The generic shape generation unit can further be configured to determine a lesion point of the lesion representing an idealized location of the lesion. The lesion point can be located at a geometric center and/or a mass center of the lesion and/or of the generic three-dimensional shape. The lesion point can be used as a numerical piece of information describing the location of the lesion. In case multiple lesions exist, the generic shape generation unit can be configured to determine multiple lesion points respectively.

According to a further embodiment, the path determination unit comprises a mesh generation unit for generating a three-dimensional internal mesh based on the acquired medical image and/or a three-dimensional external mesh based on the acquired medical image. In particular, the internal mesh comprises outer contours of the skeleton, the organs, the blood vessels, and/or the lesion of the patient while the external mesh comprises outer contours of the skin of the patient.

The internal mesh and/or the external mesh can be a representation of the patient's body and/or parts of it in the form of point clouds, edges, three-dimensional surfaces, and/or volumes. Data of the acquired medical image are used to generate the internal mesh and/or the external mesh. Hereby, the internal mesh can comprise several parts, representing outer contours of different organs, blood vessels, the lesion, and/or other internal structures of the patient's body. In particular, the internal mesh can represent vulnerable organs or tissue of the patient which should not be penetrated by the ablation needle. The external mesh represents the outer contour of the patient's skin, thus enclosing the body of the patient. In other words, the external mesh represents structures one can see from the outside of the patient while the internal mesh represents internal parts or subparts of the patient's body. The mesh generation unit can also be configured to generate the internal mesh and/or the external mesh based on a number of acquired medical images. In other words, several medical images, for example representing slices of the patient's body, can be combined to generate the internal mesh and/or the external mesh.

According to a further embodiment, the path determination unit comprises a point cloud generation unit for generating a three-dimensional skin point cloud with a number N2 of skin points, with N2 ≥ 1. In particular, the point cloud generation unit is configured to generate the skin point cloud based on the acquired medical image.

Each of the N2 skin points represents one point of the external mesh, thus, the outer contour of the patient's skin. Therefore, the point cloud generation unit can be configured to generate the skin point cloud based on the external mesh. Hereby, the point cloud generation unit can be configured to locate the N2 skin points with a given spacing in between them, resulting in evenly spread skin points. Alternatively, the density of the N2 skin points can vary, for example, showing a higher density at locations with higher curvatures of the skin of the patient, for example at the region of the neck.

According to a further embodiment, the path determination unit comprises a path generation unit for generating N2 unfiltered paths. In particular, each of the N2 unfiltered paths connects the lesion with one of the N2 skin points.

Each of the N2 unfiltered paths can connect one of the N2 skin points with the lesion point. In case multiple lesions exist, additional unfiltered paths can be generated connecting two or more of the lesions and/or lesion points with the external mesh. Said additional unfiltered paths can enable the treatment of multiple lesions using the same path, thus minimizing the potential damage to the patient and/or reducing the treatment time.

According to a further embodiment, the path determination unit comprises a hit determination unit for detecting a collision of one or more of the N2 unfiltered paths with internal body parts of the patient, wherein the hit determination unit is configured to determine a number N3 of filtered paths comprising all of the N2 unfiltered paths showing no collision with the internal body parts, with N3 ≥ 1.

In the following, the term "collision" can be understood as a penetration or an overlapping of one of the N2 unfiltered paths with internal body parts of the patient, in other words, an intersection of one of the N2 unfiltered paths with the internal body parts. Hereby, the internal body parts can be represented using the internal mesh or be the parts of the internal mesh. Hence, collisions can be determined by detecting a collision of one or more of the N2 unfiltered paths with the internal mesh. With the internal mesh representing vulnerable organs or tissue of the patient, a collision would mean that at least one vulnerable organ or tissue of the patient would be damaged if proceeded with the respective path causing the collision. Therefore, the respective "collision path" is "excluded" and not part of the N3 filtered paths. Each of the N3 filtered paths is identical or congruent to one of the N2 unfiltered paths. In other words, the N3 filtered paths represent a selection of specific ones of the N2 unfiltered paths, wherein each one of the N3 filtered paths does not show a collision with the internal body parts of the patient and/or a collision with the internal mesh. Hereby, in particular, the hit determination unit is configured to detect a collision using a ray casting scheme. Ray casting is a graphics algorithm that uses a geometric algorithm correlated to ray tracing. Ray tracing-based algorithms operate in image order to render three-dimensional scenes to two-dimensional images. The idea behind ray casting is to trace rays from one point, for example the lesion point, and to find the closest object blocking the path of that ray.

According to a further embodiment, the path determination unit comprises a path selection unit for selecting the N1 optimal paths out of the N3 filtered paths, wherein the path selection unit is configured to select the N1 optimal paths according to their respective path length, potential damage for the patient, and/or other criteria.

Each of the N1 optimal parts is identical or congruent to one of the N3 filtered paths. In other words, the N1 optimal paths represent a selection of specific ones of the N3 filtered paths, wherein the N1 optimal paths are regarded as the most optimal ones of the N3 filtered paths regarding a lesion ablation treatment. The path determination unit can be configured to select one, two, three, or more optimal paths of the N3 filtered paths. Hereby, the path selection unit can be configured to address a specific score value to each one of the N3 filtered paths describing its degree of beneficially for performing the lesion ablation treatment. In the following, the N1 optimal paths can be chosen according to their score value. By providing more than one optimal paths, the user can be provided with the freedom to follow individual preferences and/or restrictions for performing the lesion ablation treatment. In general, the shorter a path is, the more optimal it is. Nonetheless, other criteria may be respected as well, such as the respective potential damage for the patient.

According to a further embodiment, the path determination unit comprises an output generation unit for generating a graphical output visualizing the N1 selected optimal paths.

The graphical output can include the selected N1 optimal paths, the external mesh, the internal mesh, the lesion, or other parts. The graphical output can further include the N1 optimal paths in a numerical manner, for example, in form of coordinates of the one or more of the N2 skin points correlated to the N1 optimal paths as well as angles of the N1 optimal paths. Hereby, a coordinate system based on the patient's body and/or the medical imaging unit can be applied. Additionally, or alternatively, the graphical output can comprise the N1 optimal paths in a graphical manner, thus visualizing the location and trace of the N1 optimal paths relative to other parts of the patient's body. The output generation unit can be configured to plot the graphical output to the user, for example using the graphical user interface. Alternatively, or additionally, the output generation unit can be configured to store the graphical output in a medical database, for example, together with the acquired medical image.

According to a further embodiment, the user interface comprises a graphical user interface for visualizing the graphical output to the user.

The graphical user interface can be or comprise a screen, a monitor, a printer, a plotter, a tablet, a mobile device, and/or any other device capable of visualizing the graphical output.

According to a further embodiment, the graphical output comprises a graphical illustration and/or a numerical representation of the N3 optimal paths. In particular, the numerical representation comprises a table with coordinates of one or more skin points and/or angles of the N1 optimal paths.

According to a further embodiment, the input unit is configured to receive a path chosen by the user.

The chosen path can represent one of the N1 selected optimal paths. This way, the system can provide the user with the N1 selected optimal paths among which the user can select one for performing the lesion ablation treatment. Alternatively, the system can be configured to select one of N1 selected optimal paths automatically. The lesion ablation treatment can then be performed using the path received by the user, a physician, and/or any other medical staff. The input unit can be configured to store the chosen path within a medical database.

According to a further aspect, the invention relates to a computer-implemented method for using a system for determining a number N1 of optimal paths for a lesion ablation treatment of a lesion of a patient, with N1 ≥ 1. The system comprises a medical imaging unit, a computer-implemented path determination unit, and a user interface. The method comprises: acquiring a medical image of the patient by the medical imaging unit; automatically determining the N1 optimal paths based on the acquired medical image by the path determination unit; and outputting the determined N1 optimal paths to a user of the system by the user interface.

According to a further embodiment, determining the N1 optimal paths comprises: generating a three-dimensional internal mesh based on the acquired medical image and a three-dimensional external mesh based on the acquired medical image by a mesh generation unit; generating a three-dimensional skin point cloud with a number N2 of skin points based on the external mesh by a point cloud generation unit, with N2 ≥ 1; determining a lesion point of the lesion and generating N2 unfiltered paths connecting the lesion point with the N2 skin points by a path generation unit; detecting a collision of one or more of the N2 unfiltered paths with the internal mesh by a hit determination unit; determining a number N3 of filtered paths comprising all of the N2 unfiltered paths showing no collision with the internal mesh by the hit determination unit, with N3 ≥ 1; selecting the N1 optimal paths out of the N3 filtered paths according to their respective path length, potential damage for the patient, and/or other criteria by a path selection unit; and generating a graphical output of the selected N1 optimal paths for the user by an output generation unit.

According to a further embodiment, the method further comprises performing the lesion ablation treatment based on the determined N1 optimal paths.

The lesion ablation treatment can be performed by a physician, technician, and/or any other user of the suggested system.

The embodiments and features described with reference to the system of the present invention apply mutatis mutandis to the method of the present invention.

According to a further aspect, the invention relates to a computer program product comprising instructions which, when the program is executed by a computer, cause the computer to carry out the above-described method using the above-described system.

A computer program product, such as a computer program means, may be embodied as a memory card, USB stick, CD-ROM, DVD or as a file which may be downloaded from a server in a network. For example, such a file may be provided by transferring the file comprising the computer program product from a wireless communication network.

Further possible implementations or alternative solutions of the invention also encompass combinations - that are not explicitly mentioned herein - of features described above or below with regard to the embodiments. The person skilled in the art may also add individual or isolated aspects and features to the most basic form of the invention.

Further embodiments, features and advantages of the present invention will become apparent from the subsequent description and dependent claims, taken in conjunction with the accompanying drawings, in which:
- Fig. 1: shows a schematical illustration of an embodiment of a system for determining a number N1 of optimal paths for a lesion ablation treatment;
- Fig. 2: shows an illustration of an embodiment of a medical image acquired by a medical imaging unit of the system according to Fig. 1;
- Fig. 3: shows an illustration of an embodiment of an internal mesh and an external mesh based on the medical image according to Fig. 2;
- Fig. 4: shows an illustration of an embodiment of a skin point cloud with skin points based on the external mesh according to Fig. 3;
- Fig. 5: shows an illustration of an embodiment of unfiltered rays connecting the skin points according to Fig. 4 with the lesion;
- Fig. 6: shows an illustration of an embodiment of filtered rays representing such ones of the unfiltered rays according to Fig. 5 causing no collision with the internal mesh;
- Fig. 7: shows an illustration of an embodiment of the filtered rays according to Fig. 6 together with the internal mesh;
- Fig. 8: shows an illustration of an embodiment of optimal rays representing optimal one of the filtered rays according to Fig. 7; and
- Fig. 9: shows a schematical illustration of an embodiment of method for using the system according to Fig. 1.

In the Figures, like reference numerals designate like or functionally equivalent elements, unless otherwise indicated.

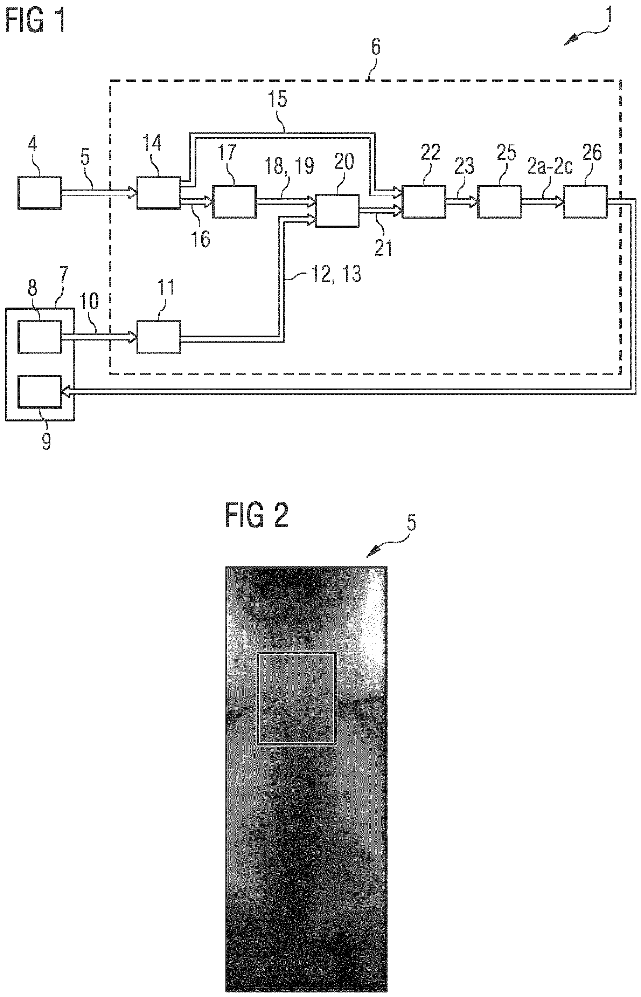

Fig. 1 shows a schematical illustration of an embodiment of a system 1 for determining a number N1 of optimal paths 2a - 2c for a lesion ablation treatment of a lesion 3 of a patient (not shown), with N1 ≥ 1. The Fig. 2 to Fig. 8 show data generated by the system 1. In the following, Fig. 1 to Fig. 8 will be referred to at the same time.

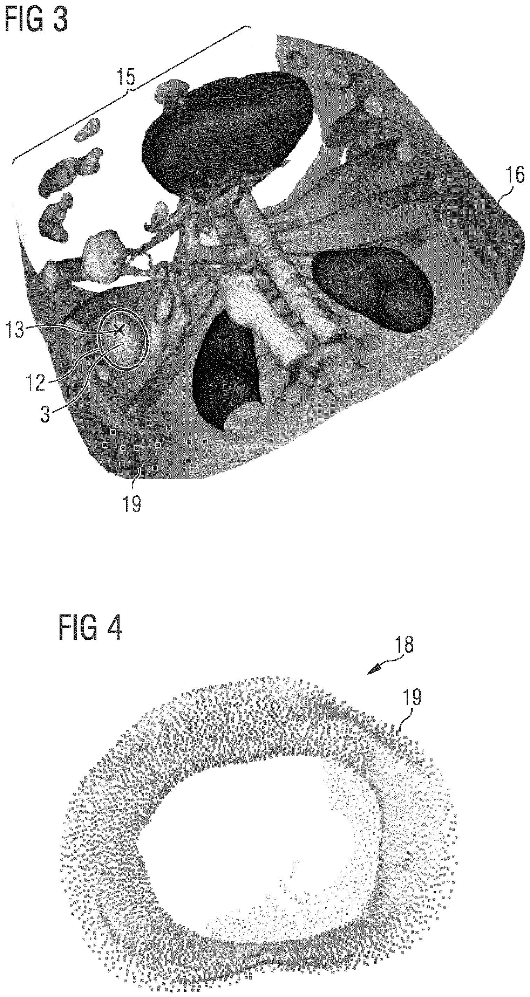

The system 1 comprises a medical imaging unit 4 for acquiring a medical image 5 (Fig. 3) of the patent. The medical imaging unit 4 can be a computer tomography [CT] unit, an X-ray unit, or any other device capable of acquiring medical images 5. The medical image 5 can be understood as an illustration of the body of the patient, a part of the body, or a slice of the body. Furthermore, the medical image 5 can comprise a number of slices through the body of the patient.

The system 1 further comprises a computer-implemented path determination unit 6 for an automatic determination of the N1 optimal paths 2a - 2c based on the acquired medical image 5. The N1 optimal paths 2a - 2c can be understood as a line, along which a technician or physician can move an ablation needle (not shown) through the body of the patient in order to place the ablation needle within the lesion 3 for performing the lesion ablation treatment, while providing the least damage to the patient, and/or the minimum path length, resulting in a reduced treatment time. The path determination unit 6 will be explained in detail below.

The system 1 further comprises a user interface 7 to enable a communication between a user (not shown) of the system 1 and the system 1. The user interface 7 comprises an input unit 8 which can be a keyboard, a mouse, a tablet display, a speech recognition system or any other system capable of receiving information or commands from the user. The user interface 7 further comprises a graphical user interface 9 which can be a monitor, a display, a printer, a plotter, a tablet display, a mobile device, or any other device capable of illustrating data to the user.

In general, tumor ablation, or percutaneous tumor ablation, comprises a range of techniques, in which the tumor tissue is destroyed using needles inserted through the skin. Therefore, a variety of types of needles can be used and placed in and/or around the lesion 3 which can be, for example, a tumor of the liver, lung, kidney, bones, or other parts of the patient. The most commonly used techniques use heat (for example radio frequency ablation or microwave ablation) or use cold (cryoablation). The technique to be used is in the following referred to as an ablation needle configuration 10.

The ablation needle configuration 10 can be determined by the user and be received by the system 1 using the input unit 8. Based on the acquired medical image 5 and the determined ablation needle configuration 10, the path determination unit 6 automatically determines the N1 optimal paths 2a - 2c, hence, without any additional effort or intervention by the user required. The graphical user interface 9 can be configured to output the determined optimal paths 2a - 2c and/or an illustration or representation of the optimal paths 2a - 2c to the user.

In the following, the subparts or subcomponents of the path determination unit 6 are described.

The path determination unit 6 comprises a generic shape generation unit 11 for generating a generic three-dimensional shape 12 (illustrated by an ellipsoid in Fig. 3) based on the received ablation needle configuration 10. The generic three-dimensional shape 12 represents an idealized impact region for the specific chosen lesion ablation treatment. The generic shape generation unit 11 is configured to determine the size and/or the shape of the generic three-dimensional shape 12 depending on the received ablation needle configuration 10 and/or the strength of the lesion ablation treatment. For example, the generic three-dimensional shape 12 can be a sphere, an ellipsoid or another three-dimensional shape. The generic three-dimensional shape 12 shall include the lesion 3 in order to ensure a proper treatment of the lesion 3. In case the lesion 3 is not entirely encapsuled by the generic three-dimensional shape 12, the user interface 7 can be configured to provide the user with a respective information, followed by a respective manipulation of the ablation needle configuration 10 by the user. In case multiple lesions 3 exist, the generic shape generation unit 11 can be configured to determine multiple generic three-dimensional shapes 12.

For every generic three-dimensional shape 12, the generic shape generation unit 11 is configured to determine a lesion point 13 (illustrated by a cross in Fig. 3) representing an idealized location of the lesion 3 in a numerical manner, for example, using a respective coordinate system. For example, the lesion point 13 can be located at a geometrical center and/or a mass center of the lesion 3 and/or of the generic three-dimensional shape 12.

The path determination unit 6 further comprises a mesh generation unit 14 for generating a three-dimensional internal mesh 15 and a three-dimensional external mesh 16 (Fig. 3). Both the internal mesh 15 and the external mesh 16 are derived from the medical image 5 or several medical images 5 and illustrate parts of the body of the patient as three-dimensional surfaces or volumes. The internal mesh 15 represents internal parts of the patient, for example, the lung, the heart, the kidneys, the skeleton, and/or the lesion 3. In particular, the internal mesh comprises vulnerable organs or tissue of the patient which should not be penetrated by the ablation needle. The external mesh 16 represents the outer contour of the skin of the patient.

The path determination unit 6 further comprises a point cloud generation unit 17 for generating a three-dimensional skin point cloud 18 with a number N2 of skin points 19, with N2 ≥ 1 (Figs. 3 and 4). Note that for illustration reasons, in Fig. 3, only a limited number of skin points 19 are visible and only one of them is referred to with a reference sign. In contrast, Fig. 4 shows the skin point cloud 18 with all exemplary N2 skin points 19. The number N2 of skin points 19 is arbitrary.

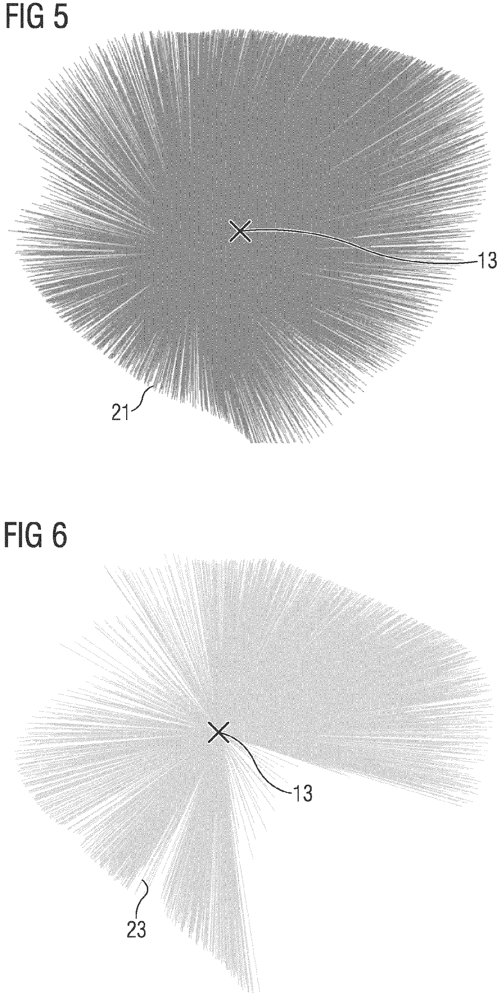

The path determination unit 6 further comprises a path generation unit 20 for generating N2 unfiltered paths 21 (Fig. 5, note that for illustration reasons, only one of the N2 unfiltered paths 21 is referred to with a reference sign). The term "unfiltered" highlights that for each of the N2 skin points 19, one unfiltered path 21 is generated. Hereby, each one of the N2 unfiltered paths 21 connects one of the N2 skin points 19 with the lesion point 13. Each of the N2 unfiltered paths 21 can be a straight line, a curved line, and/or any other shape suitable for lesion ablation treatment. In case multiple lesions 3 exist, additional unfiltered paths 21 can be generated connecting two or more of the lesions 3 and/or lesion points 13 with the external mesh 16, enabling the treatment of multiple lesions 3 using the same unfiltered path 21, thus minimizing the damage to the patient and/or minimizing treatment time.

The path determination unit 6 further comprises a hit determination unit 22 for detecting a possible collision of one or more of the N2 unfiltered paths 21 with internal body parts, in particular, with the internal mesh 15. In the following, the term "collision" can be understood as a penetration or overlapping of one of the N2 unfiltered paths 21 with one or more parts of the internal mesh 15. Since the internal mesh 15 comprises vulnerable organs or tissues of the patient, a respective collision would result in at least one of the vulnerable organs or tissues of the patient being penetrated by the ablation needle. Therefore, the respective unfiltered paths 21 showing collisions have to be filtered out and cannot be chosen for performing the ablation treatment. The hit determination unit 22 can furthermore be configured to detect a collision between the N2 unfiltered paths 21 and parts of the internal mesh 15 while respecting a given diameter of the ablation needle based on the ablation needle configuration 10.

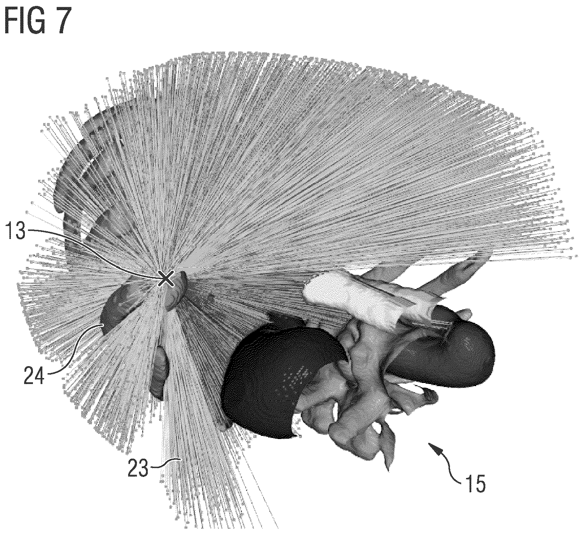

The hit determination unit 22 is configured to detect a collision using a ray casting scheme. Furthermore, the hit determination unit 22 is configured to determine a number N3 of filtered paths 23 comprising all of the N2 unfiltered paths 21 showing no collision with the internal mesh 15, with N3 ≥ 1. Hence, the N3 filtered paths 23 represent all paths along which the ablation needle can be moved through the body of the patient while not violating vulnerable organs or issues of the patient. Fig. 6 shows all of the N3 filtered paths 23. Due to illustration reasons, only one of the N3 filtered paths 23 is referred to with a reference sign. The N3 filtered paths 23 are also illustrated in Fig. 7 together with the internal mesh 15. In this way, Fig. 7 illustrates that the N3 filtered paths 23 comprise only such ones of the N2 unfiltered paths 21 which do not collide with parts of the internal mesh 15. Note, for example, that the N3 filtered paths 23 "bypass" the patient's ribs 24 at the left side of Fig. 7 in order to avoid a collision of the ablation needle with one of the ribs 24.

The path determination unit 6 can further comprise a path selection unit 25. The path selection unit 25 is configured to evaluate each of the N3 filtered paths 23 according to their specific path length, corresponding potential damage to the patient's body, treatment time, and/or other factors. For example, the path selection unit 25 can be configured to address each of the N3 filtered paths 23 with a specific score in a numerical manner defining how beneficial the specific one of the N3 filtered paths 23 is in order to perform the lesion ablation treatment.

Eventually, the path selection unit 25 is configured to define the N1 optimal paths 2a - 2c out of the N3 filtered paths 23 showing the highest scores, hence, representing the most beneficial solution for performing the lesion ablation treatment. In principle, the shorter a specific one of the N3 filtered paths 23 is, the more ideal the respective solution is due to a lower potential damage to the patient and/or a reduced treatment time.

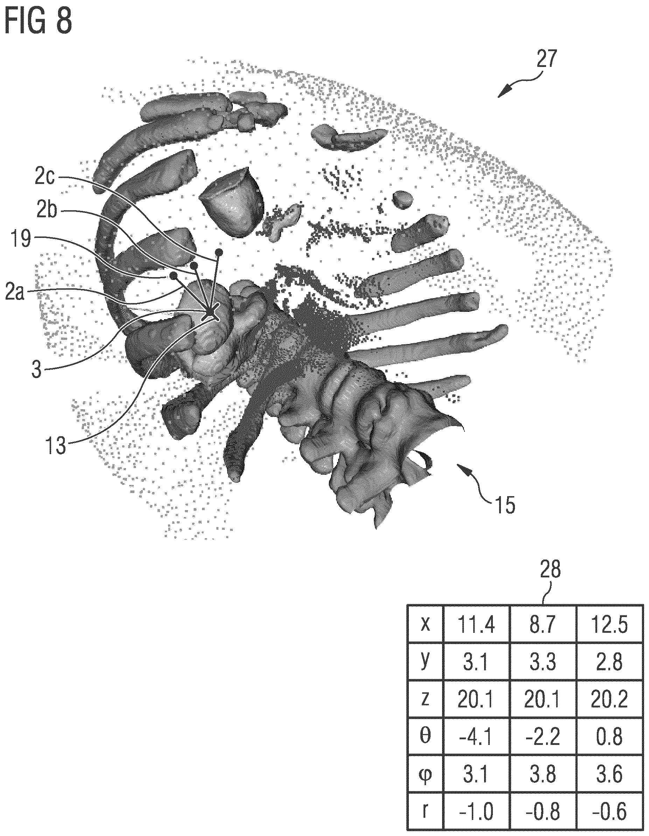

The path determination unit 6 can furthermore comprise an output generation unit 26 for generating a graphical output 27 (Fig. 8) visualizing the N1 selected optimal paths 2a - 2c in a graphical manner, hence in combination with and relative to parts of the internal mesh 15, the lesion 3, the skin point cloud 18, and/or the external mesh 16. Alternatively, or additionally, the graphical output 27 can visualize the N1 selected optimal paths 2a - 2c in a numerical manner, for example, using a table 28 comprising coordinates of the respective entry points for the ablation needle (the respective one of the N2 skin points 19 correlated to the N1 optimal paths 2a - 2c) and one or more angles indicating the direction of the respective optimal paths 2a - 2c from the respective skin point 19 to the lesion point 13.

The graphical output 27 can be stored in a medical database, for example, together with the acquired medical image 5. Alternatively, or additionally, the output generation unit 26 can be configured to visualize the graphical output 27 to the user using the graphical user interface 9. Among the N1 optimal paths 2a - 2c, the user can choose one optimal path 2a - 2c for proceeding with the ablation treatment. Hereby, the input unit 8 can be configured to receive the respective choice from the user.

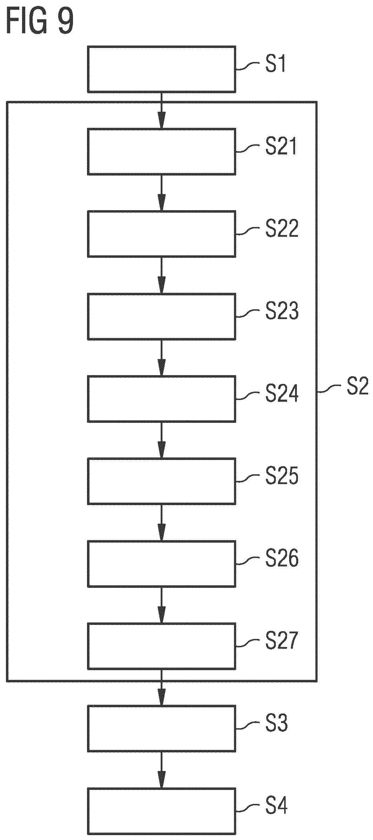

Fig. 9 shows a schematical illustration of an embodiment of a method for using the system 1.

In a first step S1, the medical image 5 is acquired by the medical imaging unit 4. In a further step S2, the N1 optimal paths 2a - 2c are automatically determined based on the acquired medical image 5 by the path determination unit 6. Hereby, the step S2 comprises a number of sub-steps which are explained in detail below. As a further main step S3, the determined N1 optimal paths 2a - 2c are outputted to the user of the system 1 by the user interface 7. Optionally, in a further main step S4, the lesion ablation treatment is performed by a physician and/or technician following the chosen one of the N1 optimal paths 2a - 2c.

In the following, the sub-steps of step S2 (determining the N1 optimal paths 2a - 2c) are explained.

In a first sub-step S21, the three-dimensional internal mesh 15 as well as the three-dimensional external mesh 16 are generated both based on the acquired medical image 5 by the mesh generation unit 14. In a further sub-step S22, the three-dimensional skin point could 18 with the N2 skin points 19 is generated based on the external mesh 16 by the point cloud generation unit 17. In a further step S23, the lesion point 13 of the lesion 3 is determined and the N2 unfiltered paths 21 are generated by the path generation unit 20. Hereby, each of the N2 unfiltered paths 21 connects one of the N2 skin points 19 with the lesion point 13. In a further step S24, a collision of one or more of the N2 unfiltered paths 21 with the internal mesh 15 is detected by the hit determination unit 22. In a further step S25, the N3 filtered paths 23 comprising all of the N2 unfiltered paths 21 showing no collision with the internal mesh 15 are determined by the hit determination unit 22. In a further step S26, the N1 optimal paths 2a - 2c out of the N3 filtered paths 23 are selected by the path selection unit 25 according to their respective path length, potential damage for the patient, and/or other criteria. In a further step S27, the graphical output 27, visualizing the N1 optimal paths 2a - 2c, is visualized to the user by the output generation unit 26.

Although the present invention has been described in accordance with preferred embodiments, it is obvious for the person skilled in the art that modifications are possible in all embodiments.

### REFERENCE NUMERALS

- 1: system
- 2a: optimal path
- 2b: optimal path
- 2c: optimal path
- 3: lesion
- 4: medical imaging unit
- 5: medical image
- 6: path determination unit
- 7: user interface
- 8: input unit
- 9: graphical user interface
- 10: ablation needle configuration
- 11: generic shape generation unit
- 12: generic three-dimensional shape
- 13: lesion point
- 14: mesh generation unit
- 15: internal mesh
- 16: external mesh
- 17: point cloud generation unit
- 18: skin point cloud
- 19: skin point
- 20: path generation unit
- 21: unfiltered path
- 22: hit determination unit
- 23: filtered path
- 24: rib
- 25: path selection unit
- 26: output generation unit
- 27: graphical output
- 28: table

## Claims

1. A system (1) for determining a number N1 of optimal paths (2a - 2c) for a lesion ablation treatment of a lesion (3) of a patient, with N1 ≥ 1, the system (1) comprising:
a medical imaging unit (4) for acquiring a medical image (5) of the patient;
a computer-implemented path determination unit (6) for an automatic determination of the N1 optimal paths (2a - 2c) based on the acquired medical image (5); and
a user interface (7) for outputting the determined N1 optimal paths (2a - 2c) to a user of the system (1).

2. The system according to claim 1,
wherein the user interface (7) comprises an input unit (8) which is configure to receive an ablation needle configuration (10) from the user, wherein the path determination unit (6) further comprises a generic shape generation unit (11) for generating a generic three-dimensional shape (12) based on the received ablation needle configuration (10), in particular, the generic three-dimensional shape (12) represents an idealized impact region for the lesion ablation treatment, wherein the generic shape generation unit (11) is configured to determine the size and/or the shape of the generic three-dimensional shape (12) depending on the received ablation needle configuration (10).

3. The system according to claim 1 or 2,
wherein the path determination unit (6) comprises a mesh generation unit (14) for generating a three-dimensional internal mesh (15) based on the acquired medical image (5) and/or a three-dimensional external mesh (16) based on the acquired medical image (5), in particular, the internal mesh (15) comprises outer contours of the skeleton, the organs, blood vessels, and/or the lesion (3) of the patient while the external mesh (16) comprises outer contours of the skin of the patient.

4. The system according to one of claims 1 - 3,
wherein the path determination unit (6) comprises a point cloud generation unit (17) for generating a three-dimensional skin point cloud (18) with a number N2 of skin points (19), with N2 ≥ 1, in particular, the point cloud generation unit (17) is configured to generate the skin point cloud (18) based on the acquired medical image (5).

5. The system according to claim 4,
wherein the path determination unit (6) comprises a path generation unit (20) for generating N2 unfiltered paths (21), in particular, each of the N2 unfiltered paths (21) connects the lesion (3) with one of the N2 skin points (19).

6. The system according to claim 5,
wherein the path determination unit (6) comprises a hit determination unit (22) for detecting a collision of one or more of the N2 unfiltered paths (21) with internal body parts of the patient, wherein the hit determination unit (22) is configured to determine a number N3 of filtered paths (23) comprising all of the N2 unfiltered paths (21) showing no collision with the internal body parts, with N3 ≥ 1.

7. The system according to claim 6,
wherein the path determination unit (6) comprises a path selection unit (25) for selecting the N1 optimal paths (2a - 2c) out of the N3 filtered paths (23), wherein the path selection unit (25) is configured to select the N1 optimal paths (2a - 2c) according to their respective path length, potential damage for the patient, and/or other criteria.

8. The system according to claim 7,
wherein the path determination unit (6) comprises an output generation unit (26) for generating a graphical output (27) visualizing the N1 selected optimal paths (2a - 2c).

9. The system according to claim 8,
wherein the user interface (7) comprises a graphical user interface (9) for visualizing the graphical output (27) to the user.

10. The system according to claim 9,
wherein the graphical output (27) comprises a graphical illustration and/or a numerical representation of the N3 optimal paths (2a - 2c); in particular, the numerical representation comprises a table (28) with coordinates of one or more skin points (19) and/or angles of the N1 optimal paths.

11. The system according to one of claims 1 - 10,
wherein the input unit (8) is configured to receive a path chosen by the user.

12. A computer-implemented method for using a system (1) for determining a number N1 of optimal paths (2A - 2C) for a lesion ablation treatment of a lesion (3) of a patient, the system (1) comprising a medical imaging unit (4), a computer-implemented path determination unit (6), and a user interface (7); the method comprising:
a) acquiring (S1) a medical image (5) of the patient by the medical imaging unit (4);
b) automatically determining (S2) the N1 optimal paths (2a - 2c) based on the acquired medical image (5) by the path determination unit (6); and
c) outputting (S3) the determined N1 optimal paths (2a - 2c) to a user of the system (1) by the user interface (7).

13. The method according to claim 12,
wherein the step b) comprises:
b1) generating (S21) a three-dimensional internal mesh (15) based on the acquired medical image (5) and a three-dimensional external mesh (16) based on the acquired medical image (5) by a mesh generation unit (14);
b2) generating (S22) a three-dimensional skin point cloud (18) with a number N2 of skin points (19) based on the external mesh (16) by a point cloud generation unit (17), with N2 ≥ 1;
b3) determining (S23) a lesion point (13) of the lesion (3) and generating N2 unfiltered paths (21) connecting the lesion point (13) with the N2 skin points (19) by a path generation unit (20);
b4) detecting (S24) a collision of one or more of the N2 unfiltered paths (21) with the internal mesh (15) by a hit determination unit (22);
b5) determining (S25) a number N3 of filtered paths (23) comprising all of the N2 unfiltered paths (21) showing no collision with the internal mesh (15) by the hit determination unit (22), with N3 ≥ 1;
b6) selecting (S26) the N1 optimal paths (2a - 2c) out of the N3 filtered paths (23) according to their respective path length, potential damage for the patient, and/or other criteria by a path selection unit (25); and
b7) generating (S27) a graphical output (27) of the selected N1 optimal paths (2a - 2c) for the user by an output generation unit (26).

14. The method according to claim 12 or 13, after step c) further comprising performing (S4) the lesion ablation treatment based on the determined N1 optimal paths (2a - 2c).

15. A computer program product comprising instructions which, when the program is executed by a computer, cause the computer to carry out the method of one of claims 12 - 14 using the system (1) of one of claims 1 - 11.
